# EUROPEAN PATENT APPLICATION

(11) **EP 2 116 200 A1**
(43) Date of publication of application: **11.11.2009**
(21) Application number: 08008536.8
(22) Date of filing: 06.05.2008
(51) Int. Cl.: A61B 17/22

(54) **Combi-device for urology and lithotripsy**

(71) Applicant: Dornier MedTech Systems GmbH, 82234 Wessling (DE)
(72) Inventor: Artmeier, Theo, 82194 Gröbenzell (DE)
(74) Representative: Grünecker, Kinkeldey, Stockmair & Schwanhäusser Anwaltssozietät

(57) **Abstract**

A combi-device (1) for urology and lithotripsy is disclosed, having an x-ray apparatus with a C-arc (4), a lithotripsy unit with a shockwave source (5) and a patient table (3), wherein the C-arc (4) can be tilted about a common pivot axis (K1) simultaneously with the patient table (3) as well as independently therefrom. The positioning opportunities and the possibilities for individual adjustment of the components of the device with respect to each other are improved so that the combi-device (1) covers a greater range of application and nevertheless permits an exact and accurate coupling of these single components. This object is achieved by a combi-device in which the patient table (3) and the C-arc (4) are separately supported.

## Description

The present invention relates to a combi-device for urology and lithotripsy with the features of the preamble of claim 1 and to a method for using this device with the features of claim 22.

The demands urology and lithotripsy make on a working environment and the corresponding necessary technical devices as, for example an x-ray device, a patient table and a lithotripsy unit, are definitely very different.

Therefore, up to now, a separate urological table with an x-ray device or a separate lithotripsy workplace, having also an x-ray device, were used. In order to save the costs for the second, redundant x-ray device, modularly composed working places were developed, comprising single devices as, for example, a separate x-ray device and a separate lithotripsy unit capable of being coupled to one another. However, coupling the separate devices to one another exactly and accurately is technically involved and in practical use, error-prone.

Trends have changed to devices and work places that combine several devices like, for example, a C-arc and a lithotripter, and that better satisfied the demands of different subareas of medicine, as, for example, urology and lithotripsy.

US 5,044,354 describes a medical device having an x-ray C-arc, a lithotripsy unit with a shockwave source and a patient bed. The shockwave source, the C-arc and the patient bed are supported on a common shaft and are pivotable about this pivot axis synchronously. By dependent separate switching of the driving motors, amongst other motions, the concurrent pivoting of the patient bed and the C-arc, the patient bed and the shockwave source, or of all three devices, can be achieved. Alternatively, the single devices can be pivoted about the common pivot axis separately. However, in clinical practice demands, that is, positioning possibilities of the single devices with respect to each other arise, which are not satisfied by the above-described workplace.

It is therefore the object of the invention to improve a combi-device of the above-mentioned kind such that the positioning opportunities and the possibilities for individual adjustment of the components of the device with respect to each other are improved so that the combi-device covers a greater range of application and nevertheless permits an exact and accurate coupling of the single components.

This object is achieved by a combi-device having the features of claim 1.

The separate support of the patient table and the C-arc, eventually also of the shockwave source, allows a greater mobility of the single components of the combi-device also with respect to each other. This permits more positioning opportunities and individual adjustment possibilities of the components relative to each other and thus a greater application range of the combi-device in different areas of medicine as, for example, urology and lithotripsy. Despite the separate support of the single components, the combi-device at the same time provides a good and rather accurate positioning of the components relative to each other. By synchronously pivoting the C-arc and the patient table about a common pivoting axis, relative movements between the C-arc and the patient table are avoided. The relative position the C-arc takes with respect to the patient table is thus kept the same during the whole pivoting process. However, prior to as well as after synchronous pivoting of the C-arc and the patient table both components can be pivoted and moved independent from each other. Nevertheless, the single components of the combi-device are separately moveable with respect to each other, due to their separate mounting. Besides this, the separate mounting of the single components of the combi-device permits a lighter construction of the combi-device by allowing the carriers of the single components to be smaller dimensioned. This helps to reduce weight inertial forces and thus to save material and costs.

Advantageously, the C-arc can be pivotable about at least one further pivot axis, being different from the pivot axis of the patient table. This allows the C-arc to be moved independently from the patient table and increases the positioning and adjustment possibilities of the C-arc in respect to the patient table.

In an embodiment of the invention, the pivot axis of the C-arc can run approximately parallel to the pivot axis of the patient table, thereby increasing the positioning opportunities of the C-arc.

Favourably, the pivot axis of the C-arc can run transverse to the pivot axis of the patient table. This permits the C-arc to be tilted or pivoted even if its pivoting axis does not align with or runs parallel to the pivot axis of the patient table, for example, when the C-arc takes a parking position outside the area of the patient table.

Further, the pivot axis of the C-arc can be moveable in a horizontal direction together with the C-arc, thereby further increasing the positioning and adjustment opportunities of the C-arc with respect to the patient table.

In an embodiment of the invention, the pivot axis of the C-arc can be pivotable about a vertical axis together with the C-arc. This allows the C-arc to be pivoted in nearly any arbitrary position it may take.

Favourably, the pivot angle about the vertical axis is in a range of 90° to 180°, preferably in a range of 110° to 160°, and particularly in a range of 120° to 140°, thereby permitting the C-arc and its pivot axis to be pivoted away from the patient table and other components of the combi-device, e.g. a shock wave source for example into a parking position.

Further, the C-arc can be translatorily moveable with respect to the patient table. This further increases the positioning possibilities of the C-arc with respect to the patient table.

In an advantageous embodiment of the invention, the C-arc can be moveable in a vertical direction relative to the patient table. This allows a height adjustment of the C-arc relative to the patient table.

Additionally, the C-arc can be moveable in a horizontal direction relative to the patient table. This further increases the adjustment possibilities of the single components relative to each other and, for example, allows the C-arc to be moved along the patient table.

In a favourable embodiment of the invention, the C-arc can be translatorily moveable relative to the shockwave source. This also helps to increase the positioning and adjustment possibilities of the single components of the combi-device with respect to each other.

Advantageously, the C-arc can be moveable relative to the shockwave source in a vertical direction. This permits a height adjustment of the C-arc relative to the shockwave source.

Further, the C-arc can be moveable in a horizontal direction relative to the shockwave source, thereby further increasing the positioning and adjustment possibilities of the C-arc relative to the shockwave source.

In addition, the C-arc can be brought into a parking position, in which the C-arc is located particularly outside the application area of the shockwave source. This frees the space around the patient table and thus increases the moveability and thus the adjustment possibilities of the shockwave source and the patient table relative to each other.

Advantageously, the C-arc can be pivotable about its pivot axis about 180° when it is in its parking position, so that the position of the image intensifier and the position of an x-ray source are interchanged. This allows the C-arc to be used in different areas of medicine as, for example, in lithotripsy by having the image intensifier place above the x-ray source as well as for urology by having the x-ray source place above the image intensifier.

In a further embodiment of the invention, a positioning mechanism for the shockwave source of a lithotripsy unit can be located beneath the patient table. This improves the subjecting feeling particularly of anxious patients. Further, a good accessibility of the patient by the physician, for example, for possible necessary reanimation, is provided.

Advantageously, the shockwave source of the lithotripsy unit can be brought into a parking position in which the shockwave source is located particularly outside the application area of the C-arc. This allows a rather unhindered pivoting or tilting of the C-arc about a pivot axis and is particularly favourable by pivoting about a great angle of rotation, e.g. 90°.

In a favourable embodiment of the invention, the shockwave source can comprise an orbital movement angle greater than 180°, when the shockwave source takes an operating position. This helps to increase the adjustment and positioning possibilities of the single components of the combi-device relative to each other and to increase the application range of the shockwave source at the patient's body.

Additionally, the orbital movement angle of the shockwave source can be at least about 200°, preferably at least about 230° and particularly at least about 260°, thereby further increasing the adjustment and application range of the shockwave source and thus of the whole combi-device.

In a further embodiment of the invention, the shockwave source can be guided isocentrically with respect to its focus, when the shockwave source takes an operating position. This allows good and easy adjustment of the shockwave source during lithotripsy treatment and also eases the handling of the shockwave source.

Favourably, a translateral movement of the patient table 3 in a vertical direction can composed of two rotary movements. This allows a spatially advantageous arrangement of the patient table and its support structure with respect to the remaining components of the combi-device and permits nevertheless a good translateral movement of the patient table.

The object is further attained with the features of claim 22.

By pivoting the patient table and the C-arc synchronously about a common axis the C-arc can maintain its relative position with respect to the patient table during the rotation or pivoting process, which is particularly favourable in urology. Nevertheless, the separate support of the C-arc and the patient table of the combi-device also allows separate movements of the C-arc and the patient table independent of each other, thereby increasing adjustment and positioning possibilities of these single components with respect to each other.

Advantageously a pivot axis of the C-arc and the pivot axis of the patient table are brought into alignment, such that the pivot axis of the C-arc coincides with the pivot axis of the patient table before synchronous pivoting of the C-arc and the patient table, thereby avoiding relative movements of the C-arc and the patient table during synchronous pivoting.

In the following, an embodiment of the invention is described, based on the following drawing.
- Figure 1: shows a perspective view of the inventive combi-device with the C-arc and the shockwave source taking a parking position,
- Figure 2: shows the combi-device of Figure 1 with the C-arc being in a parking position and performing a 180° rotation about its pivot axis,
- Figure 3: shows the combi-device prepared for urology treatment with the C-arc taking an operating position and the shockwave source being in its parking position,
- Figure 4: shows the inventive combi-device while x-ray scanning of a patient,
- Figure 5: shows the inventive combi-device with the patient table taking a 90° micturition position for urological treatment and the C-arc taking an x-ray scan of the patient,

- Figure 6: shows the inventive combi-device for a lithotripsy treatment, and
- Figures 6a to: 6e show the orbital movement angle of the shockwave source when it takes its operating position.

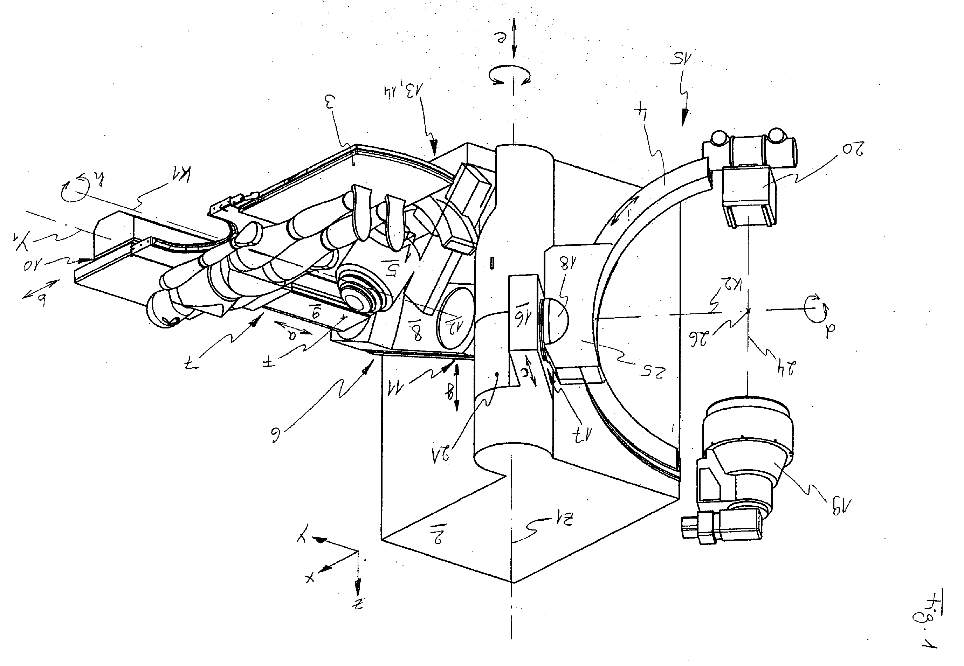

Figure 1 shows a perspective view of a first embodiment of the inventive combi-device 1. It has a base unit 2 on which the patient table 3, an x-ray C-arc 4 and a shockwave source 5 are arranged.

In the following, the inventive design of the combi-device is described with a reference to an orthogonal system having X-, Y- and Z-axes. In this context, the X-axis runs horizontally with respect to the floor on which the base unit 2 is placed and along side the patient table 3 when it extends parallel to the floor, as indicated in Figure 1. The Y-axis runs also horizontally with respect to the floor that is parallel thereto, but perpendicular to the X-axis and the Z-axis runs in a vertical direction with respect to the floor on which the base unit 2 stands, as indicated in Figure 1.

The patient table 3 is supported on the base 2 by a cantilever 6 and can be moved in X, Y and Z directions. Further, the patient table 3 is tiltable about a pivoting axis K1.

In this embodiment, the cantilever 6 is L-shaped, having a first arm 7 extending in Y direction and a second arm 8 running perpendicular thereto. The patient table 3 is arranged on the first arm 7 comprising two linear guides 9, 10. The first linear guide 9 guides the patient table 3 along the Y-axis closer to the base unit 2 or farther therefrom, as indicated by the double arrow a. The second linear guide 10 allows the patient table 3 to be moved in X direction, along side the base unit 2 as indicated by the double arrow b.

The cantilever 6, that is the second arm 8 thereof, is supported on the base 2 by a linear guide 11, which permits movement of the cantilever and thus the patient table 3 supported thereon in Z direction, as indicated by double arrow g. In the present embodiment the linear guide 11 is designed as a slide or carriage. However, in alternative embodiments of the invention the linear motion of the patient table 3 in Z direction can also be accomplished by different means known in the art. The second arm 8 is attached to the slide of the linear guide 11 by a swivel joint 12, allowing the second arm 8 of the L-shape cantilever 6 to be pivoted about the pivot axis K1 of the patient table 3, as indicated by double arrow h.

Naturally, nearly all possible spatial movements of the patient table 3 can be achieved by arbitrarily combining the different motion opportunities of the cantilever 6.

In Figure 1, the shockwave source 5 is in a parking position 13. In this condition, the shockwave source 5 and its positioning mechanism 14, via which it is attached to the base unit 2, are arranged as close to the base unit 2 as possible, in order to free the space beneath and around the patient table 3.

In Figure 1, also the C-arc 4 is in a parking position 15 outside the application area of the patient table 3 and the application area of the shockwave source 5. The C-arc 4 is mounted to a console 16, which is pivotably supported on the base unit 2 by means of a swivel joint 21. The console 16 comprises a linear guide 17, by means of which the C-arc can be moved along the console 16 in the direction of double arrow c.

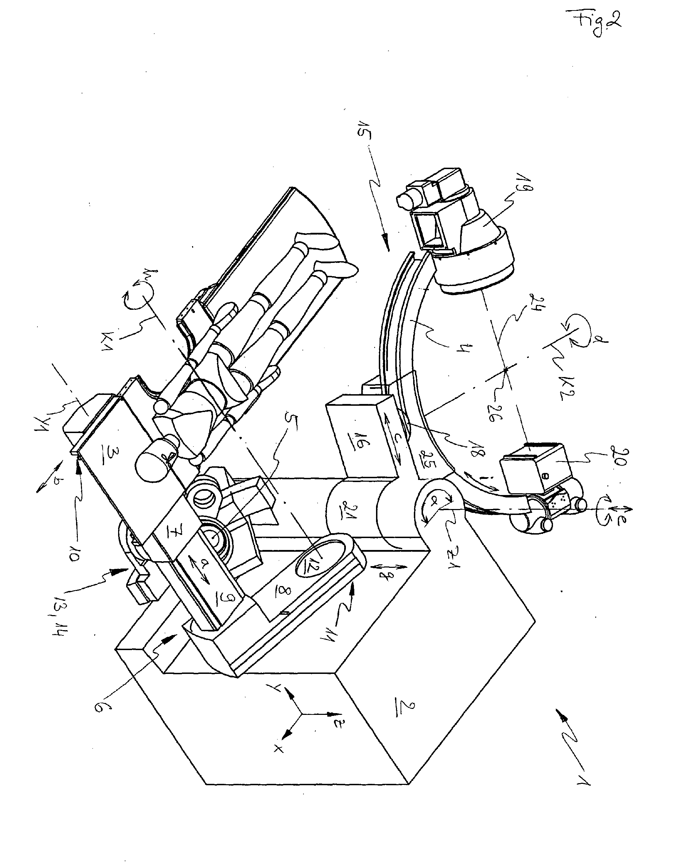

The C-arc 4 comprises a swivel joint 18 permitting the C-arc 4 to be pivoted about a pivot axis K2 in the direction of double arrow d. Further, the C-arc 4 has a guide 25 allowing the C-arc 4 to be orbitally moved in the direction of double arrow i. When the C-arc is in its parking position 15, like here, the C-arc 4 can be tilted about this pivot axis K2 about at least 180°, so that the positions of an image intensifier 19 and an x-ray source 20 located at the free ends of the C-arc 4 are interchanged, as indicated in Figure 2. In Figure 2, the same reference signs indicate the same parts, as in Figure 1.

The whole console 16 can be moved up and down the base unit 2 along the Z-axis in the direction of double arrow e.

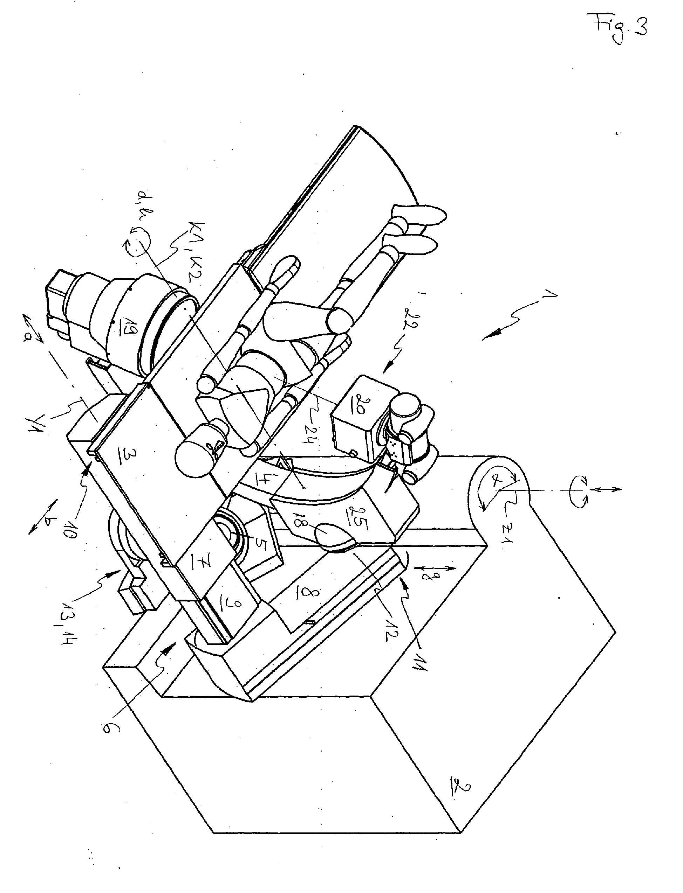

By its pivotable support, namely by swivel joint 21, the console 16 and thus the C-arc 4 can be pivoted about an axis Z1 of the base unit 2 into an operating position 22 of the C-arc 4, as shown in Figure 3.

Figure 3 shows the C-arc 4 in an operating position 22 with the console 16 being pivoted about the axis Z1 of the base unit 2, thus the direction of the linear guide 17 indicated by double arrow c coincides with or runs parallel to the X-axis. The reference signs used in Figure 3 indicate the same parts as the reference signs used in Figures 1 and 2. The description of those parts is thus omitted. In this case, the x-ray source 20 is positioned above the patient table 3 and the image intensifier 19 is positioned beneath the patient table 3 as close as possible thereto. This arrangement is particularly useful in urological examination and treatment.

The shockwave source 5 is here still in its parking position 13.

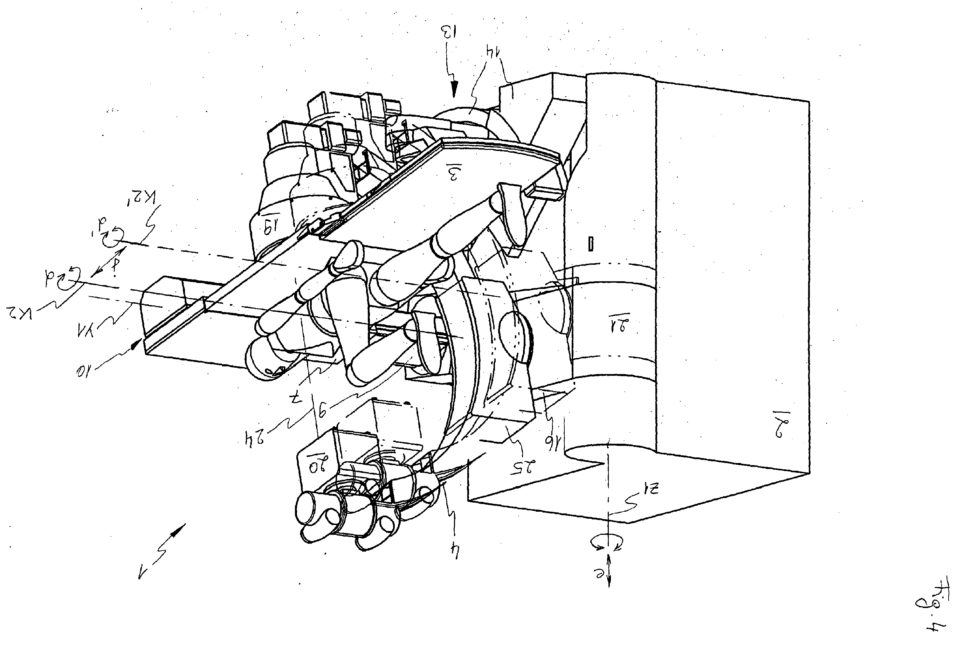

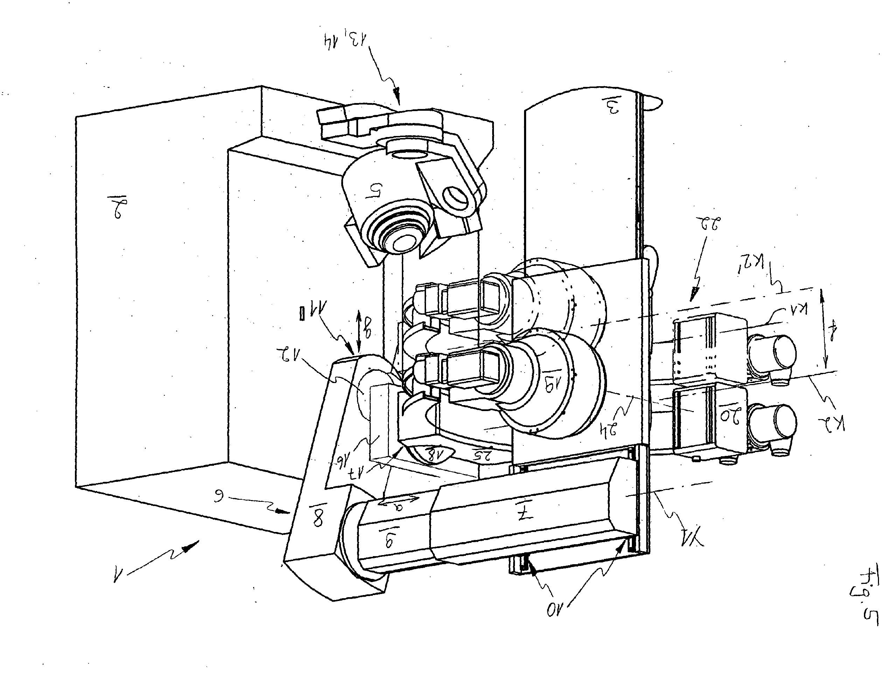

The different motion opportunities of the console 16 and the C-arc 4 described above allow an x-ray scanning of a patient in nearly any arbitrary position of the patient table 3, as indicated, for example, in Figures 4 and 5 by the dashed lines. The reference signs of Figures 4 and 5 indicate the same parts as in Figures 1 through 3 and the description of those elements is omitted.

By combining the movement of the C-arc 4 in the X direction by linear guide 17 and the movement of the console 16 in the Z direction, the C-arc 4 can be moved along the patient table in nearly any arbitrary pivoting position of the patient table 3, as shown by the solid and dashed lines in Figure 4. Thereby, also the pivot axis K2 of the C-arc 4 is shifted into a position indicated with K2', as indicated by arrow j.

By means of the cantilever 6, the patient table 3 can be pivoted about pivot axis K1 until it takes an upright position, also called a 90° or micturition position, as shown in Figure 5. This is of particular use in urological treatment. Here, too, an x-ray scanning of a patient lying on the patient table 3 is possible by moving the console 16 and thus the C-arc 4 in the Z direction along axis Z1, as indicated by arrow e as well as the solid and dashed lines. Moving the console 16 and the C-arc 4 along axis Z1 of the base unit 2 also shifts the pivot axis K2 of the C-arc 4 in this direction, namely into a position K2' as indicated by arrow f.

For simultaneous pivoting of the patient table 3 and the C-arc 4, the pivot axis K1 of the patient table 3 and the pivot axis K2 of the C-arc 4 are brought into alignment such that axis K1 coincides with axis K2. Naturally, the C-arc 4 can be moved along the patient table 3 and thus x-ray scanning of a patient can be performed in nearly any arbitrary pivoting position of the patient table 3 by concurrently moving the C-arc 4 in the X direction and moving the console 16 in the Z direction.

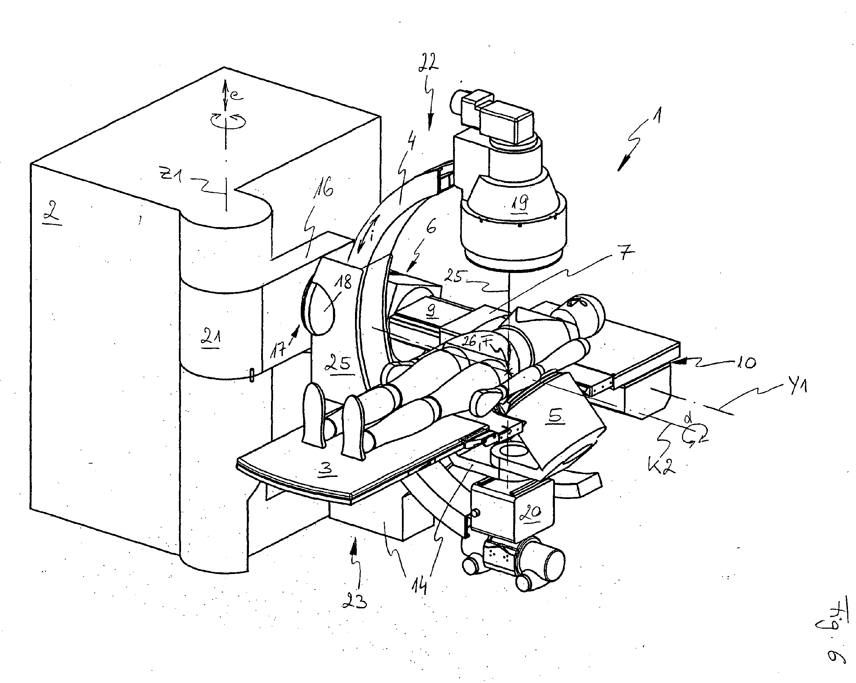

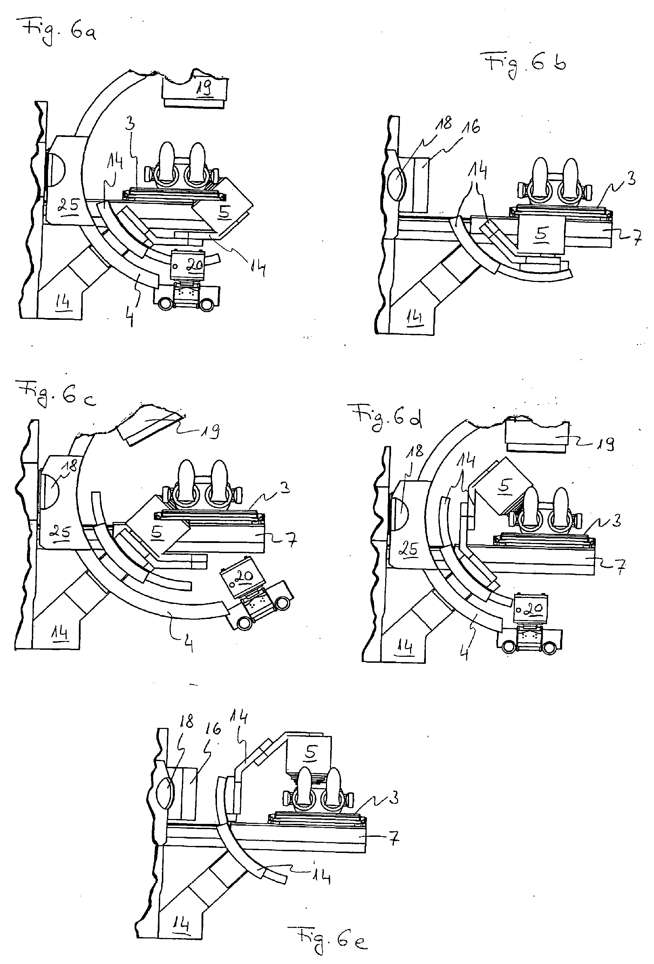

Figure 6 shows the inventive combi-device 1 for application in lithotripsy treatment. In Figure 6, the same reference signs are used as in Figures 1 through 5 and the corresponding description of those elements is thus omitted.

Here, both the C-arc 4 and the shockwave source 5 are in their operating positions 22 and 23. For lithotripsy, the image intensifier 19 is positioned above the patient table 3 and the x-ray source 20 is positioned beneath the patient table 3.

In its operating position 23, the shockwave source 5 is isocentrically guided with respect to the focus F of the shockwaves by the positioning mechanism 14. The positioning mechanism 14 is located substantially beneath the patient table 3. This frees the space above the patient table 3 and allows the physician good accessibility to a patient lying on the patient table 3. Further, the feeling of the patient can be improved, as the space directly above him is mostly comparatively free. This is especially advantageous for anxious patients.

In this embodiment, the orbital motion angle of the shockwave source 5 is 225°. This allows the shockwave source 5 to take a great range of application positions. Thus, over table positions for treating the right or left kidney as well as under table positions on both long sides of the patient table 3 are possible, as can be seen from Figures 6 a through e, showing the orbital motion angle of the shockwave source 5. The reference signs used in Figures 6 a through e indicate the same parts as in Figures 1 through 6, so that the description of those elements is omitted.

In other favourable embodiments of the invention, the orbital motion angle of the shockwave source 5 can be even greater, for example, up to 270° and more.

In the following, the function of the combi-device shown in Figures 1 through 6e is described.

When a patient enters the surgery, the combi-device 1 will most probably be in a position as shown in Figure 1, that is, with the C-arc 4 and the shockwave source 5 being in their parking positions 15 and 13, and the patient table 3 being in its lowest and outermost position as far as possible from the base unit 2. This allows the patient an easy entry onto the patient table 3.

Next, the patient will be brought to a comfortable height for the physician by movement of the cantilever 6. Therefore the cantilever 6 supporting the patient table 3 is moved in Z direction by means of the linear guide 11, causing a lifting of the patient table 3.

For a urological treatment, the C-arc 4, when it is in its parking position 15, is tilted about pivot axis K2 until the x-ray source 20 is positioned above the image intensifier 19, as indicated in Figure 2.

Then, the C-arc 4 is brought into its operating position 22 by pivoting the console 16 about axis Z1 of the base unit 2, until the linear guide 17 on console 16 runs in or parallel to the X direction.

A specific region of the patient to be x-rayed is positioned in the centre beam 24 of the x-ray C-arc 4 by moving the patient table 3 in the X direction by means of linear guide 9 and in Y direction by means of linear guide 10. Then, an x-ray picture can be taken or, alternatively, the patient can be x-ray scanned as, for example, shown in Figures 4 and 5.

In urological treatment, it can be necessary to bring the patient in a so-called 90° micturition position, as shown in Figure 5. Therefore, the patient table is tilted about pivot axis K1 by means of cantilever 6. Also, simultaneous pivoting of the C-arc 4 and the patient table 3 is possible. Therefore, the pivot axis K2 of the C-arc 4 and the pivot axis K1 of the patient table 3 are brought into alignment. The C-arc 4 and its pivot axis K2 is moved by means of the linear guide 17 on console 16 as well as by moving the console 16 itself along axis Z1 of the base unit 2, until axis K2 of the C-arc 4 coincides with the pivot axis K1 of the patient table 3. Then both, the C-arc 4 and the patient table 3, are synchronously pivoted into a desired treatment position. As the two pivot axis K1 and K2 coincide, there is no relative movement between the patient table 3 and the C-arc 4 during synchronous pivoting of these components. Thereafter, the components 3 and 4 of the combi-device can be moved independently from each other again.

Also, in the micturition position shown in Figure 5 and in nearly any arbitrary tilting position of the patient table 3, scanning of a patient is possible by moving the console 16 along axis Z1 of the base unit 2, that is, by moving the console 16 in the Z direction and/or concurrently moving the C-arc 4 along linear guide 17 in the X direction.

During urological treatments, the shockwave source 5 usually keeps its parking position 13 in order not to unnecessarily occupy space beneath the patient table 3. This allows a better and easier positioning of the C-arc 4 and the patient table 3.

When a lithotripsy treatment is to be performed, the C-arc 4 is brought into its parking position 15 outside the application area of the shockwave source 5 and the patient table 3. Therefore, the C-arc 4 is pivoted about a vertical axis of the base unit 2, namely about axis Z1 about approximately 135°. This rotation angle allows the C-arc 4 to take a good parking position 15 beside the patient table 3, in which unhindered pivoting of the C-arc 4 is possible. However, in other embodiments of the invention, this rotation angle α may vary. Basically, it can be in the range of 90° to 180°, preferably in the range of 110° to 160°, and particularly in a range of 120° to 140°.

Then, the C-arc 4 is pivoted about its pivot axis K2 about 180°. Thus, the image intensifier 19 and the x-ray source 20 interchange their position. Now, the image intensifier 19 is located above the patient table 3 and the x-ray source 20 is located beneath the patient table 3.

Then, the C-arc 4 is brought into its operating position 22 again by pivoting the console 16 about axis Z1 of the base unit 2. Also, the shockwave source 5 is brought into its operating position 23 by expanding and deploying the positioning mechanism 14. Both, the C-arc 4 and the shockwave source 5 are arranged such, that an x-ray focus 26 of the C-arc 4 coincides with the focus F of the shockwave source 5.

In order to locate a concrement in a patient's body, usually x-ray pictures are taken at two different angles. Therefore, the C-arc 4 can be tilted about its pivot axis K2 in the direction of double arrow d. Alternatively, the two different angles can be achieved by moving the C-arc 4 orbitally by means of guide 25, as indicated by double arrow i.

In taking these different positions the C-arc 4 may deform due to the weight of the x-ray source 20 and the image intensifier 19, which can result in shifting of the x-ray focus 26. This can be compensated by moving the console 16 and thus the C-arc 4 in Z direction that is along axis Z1 such that the focus F of the shockwave source 5 aligns with the x-ray focus 26 again.

Although the x-ray focus 26 of this embodiment of the invention aligns with the center of rotation of the C-arc 4 viz. with the isocenter of the C-arc 4, in alternative embodiments of the invention the x-ray focus 26 can also be located differently, for example at some point along the central x-ray beam 24 of the C-arc 4 or even apart therefrom.

Once the concrement is located, it is brought into alignment with the focus F of the shockwave source 5 by moving the patient table 3 in X-, Y- and Z- direction by means of the cantilever 6 and the linear guides 9, 10 and 11. In order to find a good window in the patient's body, the shockwaves can pass unhinderedly and also, to vary the skin area through which the shockwaves are applied, it may be necessary to move the shockwave source 5 around the patient. The positioning mechanism 14 allows the shockwave source 5 to take a lot of different application positions and guides the shockwave source 5 on a spherical plane with respect to its focus F. The shockwave source 5 thus can nearly take any position on this spherical plane except the positions blocked by the patient table 3 and positions not reachable due to the limited orbital motion angle of the shockwave source 5.

When lithotripsy treatment is finished, the shockwave source 5 and the C-arc 4 can be brought into their parking positions 13, 15 again in order to have them out of the way and the patient table 3 can be lowered in Z direction by linear guide 11 and moved away from the base unit 2 by linear guide 9, as shown in Figure 1. This facilitates the patient getting off the patient table.

The above-described inventive combi-device is modularly designed. This means that according to the demands of a special application or to the desires of a particular client, the combi-device can easily be adapted to a mere lithotripsy workplace or a mere urological workplace.

For downgrading the above-described modular combi-device 1 of full expansion stage to a mere lithotripsy workplace, the moveability of the C-arc 4 in the Z as well as in the X direction are omitted. Regarding the above-described embodiment, this means that the swivel joint 21 for moving the console 16 about axis Z1 and the linear guide 17 for moving the C-arc 4 in the X direction when it is in its operating position 22 are omitted. This results in the C-arc 4 being still tiltable about its pivot axis K2 in the direction of double arrow d as well as being orbitally moveable in the direction of double arrow i, but having no further motion possibilities. Thus, x-ray pictures at two or more different pivot angles or at two or more orbital angles can still be taken and a concrement can be thus located in a patient's body. The concrement can then be treated with shockwaves by means of the shockwave source 5.

For downgrading the above-described inventive combi-device 1 of full expansion stage to a mere urological workplace, the shockwave source 5 and its positioning mechanism 14 can be omitted. Thus the C-arc maintains completely its above-described motion opportunities and still allows the taking of x-ray pictures as well as x-ray scanning of the patient in a lot of different positions.

## Claims

1. A combi-device (1) for urology and lithotripsy having an x-ray apparatus with a C-arc (4), a lithotripsy unit with a shockwave source (5) and a patient table (3), wherein the C-arc (4) can be tilted about a common pivot axis (K1) simultaneously with the patient table (3) as well as independently therefrom, **characterized in that** the patient table (3) and the C-arc (4) are separately supported.

2. The combi-device (1) according to claim 1, **characterized in that** the C-arc (4) is pivotable about at least one further pivot axis (K2) being different from the pivot axis (K1) of the patient table (3).

3. The combi-device (1) according to claim 2, **characterized in that** the pivot axis (K2) of the C-arc (4) runs approximately parallel to the pivot axis (K1) of the patient table (3).

4. The combi-device (1) according to claim 2, **characterized in that** the pivot axis (K2) of the C-arc (4) runs transverse to the pivot axis (K1) of the patient table (3).

5. The combi-device (1) as claimed in at least one of the preceding claims, **characterized in that** the pivot axis (K2) of the C-arc (4) is moveable in a horizontal direction (X) together with the C-arc (4).

6. The combi-device (1) as claimed in at least one of the preceding claims, **characterized in that** the pivot axis (K2) of the C-arc (4) is capable of being pivoted about a vertical axis (Z1) together with the C-arc (4).

7. The combi-device (1) according to claim 6, **characterized in that** a pivot angle (α) of the C-arc (4) about the vertical axis (Z1) is in a range of 90° to 180°, preferably in a range of 110° to 160°, and particularly in a range of 120° to 140°.

8. The combi-device (1) according to at least one of the preceding claims, **characterized in that** the C-arc (4) is moveable translatorily with respect to the patient table (3).

9. The combi-device (1) according to at least one of the preceding claims, **characterized in that** the C-arc (4) is moveable in a vertical direction (Z) with respect to the patient table (3).

## Amended claims

### Amended claims in accordance with Rule 137(2) EPC.

**1.** A combi-device (1) for urology and lithotripsy having an x-ray apparatus with a C-arc (4) having a pivot axis (K2), a lithotripsy unit with a shockwave source (5) and a patient table (3) having a pivot axis (K1) being different from the pivot axis (K2) of the C-arc, the two pivot axes (K1, K2) providing pivot ability of the C-arc (4) and the patient table (3) about different axes, wherein the patient table (3) and the C-arc (4) are separately supported and the two pivot axes (K1, K2) are alignable to coincide with each other such that the C-arc (4) and patient table (3) can be tilted about the common pivot axes (K1, K2) simultaneously as well as independently from each other.

**2.** The combi-device (1) according to claim 1, **characterized in that** the pivot axis (K2) of the C-arc (4) runs approximately parallel to the pivot axis (K1) of the patient table (3).

**3.** The combi-device (1) according to claim 1, **characterized in that** the pivot axis (K2) of the C-arc (4) runs transverse to the pivot axis (K1) of the patient table (3).

**4.** The combi-device (1) as claimed in at least one of the preceding claims, **characterized in that** the pivot axis (K2) of the C-arc (4) is moveable in a horizontal direction (X) together with the C-arc (4).

**5.** The combi-device (1) as claimed in at least one of the preceding claims, **characterized in that** the pivot axis (K2) of the C-arc (4) is capable of being pivoted about a vertical axis (Z1) together with the C-arc (4).

**6.** The combi-device (1) according to claim 5, **characterized in that** a pivot angle (α) of the C-arc (4) about the vertical axis (Z1) is in a range of 90° to 180°, preferably in a range of 110° to 160°, and particularly in a range of 120° to 140°.

**7.** The combi-device (1) according to at least one of the preceding claims, **characterized in that** the C-arc (4) is moveable translatorily with respect to the patient table (3).

**8.** The combi-device (1) according to at least one of the preceding claims, **characterized in that** the C-arc (4) is moveable in a vertical direction (Z) with respect to the patient table (3).

**9.** The combi-device (1) according to at least one of the preceding claims, **characterized in that** the C-arc (4) is moveable in a horizontal direction (X) with respect to the patient table (3).

**10.** The combi-device (1) according to at least one of the preceding claims, **characterized in that** the C-arc (4) is translatorily moveable with respect to the shockwave source (5).

**11.** The combi-device (1) according to at least one of the preceding claims, **characterized in that** the C-arc (4) is moveable in a vertical direction (Z) with respect to the shockwave source (5).

**12.** The combi-device (1) according to at least one of the preceding claims, **characterized in that** the C-arc (4) is moveable in a horizontal direction (X) with respect to the shockwave source (5).

**13.** The combi-device (1) according to at least one of the preceding claims, **characterized in that** the C-arc (4) is capable of taking a parking position (15), in which the C-arc (4) is located particularly outside the application area of the shockwave source (5).

**14.** The combi-device (1) according to at least one of the preceding claims, **characterized in that** the C-arc (4) is capable of being tilted about its pivot axis (K2) about at least 180° when it is in its parking position (15), so that the position of an image intensifier (19) and an x-ray source (20) are interchanged.

**15.** The combi-device (1) according to at least one of the preceding claims, **characterized in that** a positioning mechanism (14) for the shockwave source (5) of the lithotripsy unit is located beneath the patient table (3).

**16.** The combi-device (1) according to at least one of the preceding claims, **characterized in that** the shockwave source (5) of the lithotripsy unit is capable of taking a parking position (13), in which the shockwave source (5) is located particularly outside the application area of the C-arc (4).

**17.** The combi-device (1) according to at least one of the preceding claims, **characterized in that** the shockwave source (5) has an orbital motion angle of more than 180°, when it takes an operating position (23).

**18.** The combi-device (1) according to claim 17, **characterized in that** the orbital motion angle of the shockwave source (5) is at least about 200°, preferably at least about 230° and particularly at least about 260°.

**19.** The combi-device (1) according to at least one of the preceding claims, **characterized in that** the shockwave source (5) is isocentrically guided by means of the positioning mechanism (14) when the shockwave source (5) takes an operating position (23).

**20.** The combi-device (1) according to at least one of the preceding claims, **characterized in that** a translateral movement of the patient table in a vertical direction (Z) is composed of two pivot movements.

**21.** Method for positioning components of a combi-device (1) for urology and lithotripsy, having an x-ray apparatus with a C-arc (4), a lithotripsy unit with a shockwave source (5) and a patient table (3), wherein the C-arc (4) and the patient table (3) are separately supported and the C-arc (4) and the patient table (3) are pivoted either synchronously or independently from each other about a common pivot axis (K1).

**22.** Method according to claim 21 **characterized in that** a pivot axis (K2) of the C-arc (4) and a pivot axis (K1) of the patient table (3) are brought into alignment such that the pivot axis (K2) of the C-arc (4) coincides with the pivot axis (K1) of the patient table (3) before synchronous pivoting of the C-arc (4) and the patient table (3).
